# EUROPEAN PATENT SPECIFICATION

(11) **EP 0 420 025 B1**
(45) Date of publication and mention of the grant of the patent: **27.12.1995**
(21) Application number: 90118038.0
(22) Date of filing: 19.09.1990
(51) Int. Cl.: C07C 37/16, C08K 5/13, C07C 39/15

(54) **Process for the preparation of phenolic antioxidants**
Herstellung von Oxydationsverhinderern
Préparation des antioxydants

(30) Priority: 25.09.1989 US 411631; 25.09.1989 US 411654
(43) Date of publication of application: 03.04.1991
(73) Proprietor: ALBEMARLE CORPORATION, Richmond, Virginia 23219 (US)
(72) Inventor: Goodard, Lloyd Edwin, Orangeburg, South Carolina 29115 (US); Mina, George Louis, Orangeburg, South Carolina 29115 (US); Heidebrecht, Gary Dean, Orangeburg, South Carolina 29115 (US)
(74) Representative: Schwabe - Sandmair - Marx

(56) References cited:
- GB-A- 894 620
- GB-A- 1 327 542

## Description

Rocklin et al. U. S. 3,026,264 describes the antioxidant use of several 3,5-dialkyl-4-hydroxybenzyl-substituted benzenes such as 2,4,6-tri(3,5-di-tert-butyl-4-hydroxybenzyl)mesitylene. They are made by the reaction of 2,6-di-alkyl-4-hydroxymethylphenols with a benzene compound in an inert solvent at -15 to 100°C in the presence of sulfuric acid or a Friedel Crafts catalyst. Shin U. S. 3,925,488 shows the same reaction except using an organic sulfonic acid catalyst and distilling water from the reaction.

Gurvich et al. G.B. 1,327,542 discloses a process for making 2,4,6-tri(3,5-dialkyl-4-hydroxybenzyl)benzenes by reacting a 2,6-dialkyl-4-methoxymethylphenol with an alkylbenzene compound in an inert solvent in the presence of an acidic catalyst such as sulfuric acid or toluene sulfonic acid. In Examples 1, 3, and 4, Gurvich et al. uses 364 parts by weight 94% sulfuric acid per mole part mesitylene and, in Example 7, 3.1 moles of toluene sulfonic acid per mole of 2,6-di-tert-butyl-4-methoxymethyl-phenol. In a commercial operation this presents a severe spent acid disposal problem. However, merely reducing the amount of acid results in a reaction wherein less than all of the reactive positions on the benzene compound become substituted. In the case of mesitylene and 2,6-di-tert-butyl-4-methoxymethylphenol, lowering the amount of sulfuric acid gives a product which contains both mono- and di-3,5-di-tert-butyl-4-hydroxybenzyl-substituted mesitylene by-products, making it unacceptable for commercial sale. Thus, a need exists for a process which allows reduction in the amount of sulfuric or sulfonic acid used as catalyst and at the same time gives a product suitable for commercial use.

It has now been discovered that the reaction of a 2,6-dialkyl-4-methoxymethylphenol with a benzene compound such as mesitylene can be effectively catalyzed using reduced amounts of sulfuric or sulfonic acid by conducting the reaction under temperature and pressure conditions that cause the methanol formed during the reaction to distill out of the reaction mixture.

A preferred embodiment of the invention is a process for making a compound having the structure: 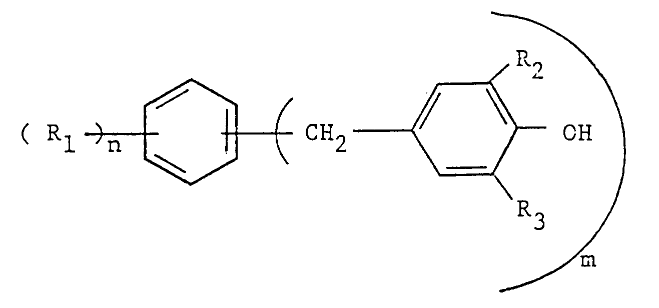
by feeding over a period of from 0.5 to 12 hours an aryl-substituted methoxy methyl reactant having the structure 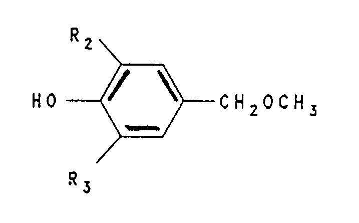
with a benzene compound having the structure 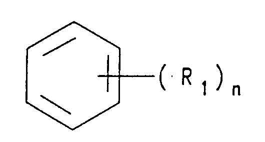
wherein R₁ is C₁₋₃ alkyl and R₂ and R₃ are independently selected from the group consisting of C₁₋₈ alkyl, C₅₋₈ cycloalkyl and C₇₋₁₂ aralkyl, n is zero or an integer from 1 to 4, m is an integer from 2 to 3, and m + n does not exceed 6, in an inert solvent in the presence of sulfuric acid or sulfonic acid at a temperature of 10-150°C and at a pressure such that the methanol formed during the reaction, distills out of the reaction mixture as a methanol-containing distillate, contacting said distillate with an absorbent having an affinity for methanol, thereby removing at least part of the methanol from said methanol-containing distillate and recycling the distillate resulting from the contact with said absorbent having an affinity for methanol.

In describing the present invention, the word "alkyl" in 2,6-dialkyl-4-methoxymethylphenol includes cycloalkyls and arylalkyls and the two alkyls on each such reactant can be the same or different.

Useful 2,6-dialkyl-4-methoxymethylphenols include:
2,6-dimethyl-4-methoxymethylphenol
2-methyl-6-tert-butyl-4-methoxymethylphenol
2,6-diisopropyl-4-methoxymethylphenol
2,6-diisobutyl-4-methoxymethylphenol
2,6-di-tert-butyl-4-methoxymethylphenol
2,6-di-sec-butyl-4-methoxymethylphenol
2-methyl-6-tert-octyl-4-methoxymethylphenol
2-methyl-6-cyclopentyl-4-methoxymethylphenol
2,6-dicyclopentyl-4-methoxymethylphenol
2,6-dicyclohexyl-4-methoxymethylphenol
2-tert-butyl-6-cyclooctyl-4-methoxymethylphenol
2,6-di-benzyl-4-methoxymethylphenol
2-methyl-6-benzyl-4-methoxymethylphenol
2,6-di-(α-methylbenzyl)-4-methoxymethylphenol
2-methyl-6-(α-methylbenzyl)-4-methoxymethylphenol
2-isopropyl-6-(α,α-dimethylbenzyl)-4-methoxymethylphenol.

The most preferred 2,6-dialkyl-4-methoxymethylphenol is 2,6-di-tert-butyl-4-methoxymethylphenol.

Suitable benzene compounds include benzene and C₁₋₃ alkyl-substituted benzenes such as toluene, m-xylene, p-xylene, durene, mesitylene, ethylbenzene, 1,3-di-ethylbenzene, 1,4-diisopropylbenzene and the like. The preferred benzene compounds are the methyl-substituted benzenes such as durene and especially mesitylene.

Useful solvents include any normally liquid material that is substantially inert under reaction conditions. Such solvents include aliphatic and cycloaliphatic hydrocarbons as well as aliphatic and aromatic halohydrocarbons. Representative examples are cyclohexane, heptane, octane, isooctane, nonane, dichloromethane, chloroform, carbon tetrachloride, 1,2-dichloroethane, dibromoethane and the like.

The most preferred inert solvents are the normally liquid aliphatic and aromatic halohydrocarbons boiling in the range of 40-200°C. More preferably the halohydrocarbon will boil in the range of 50-150°C at atmospheric pressure. Representative examples of such solvents are 1,2-dichloroethane, 1,1-dichloroethane, 1,1,1-trichloroethane, 1,1,2-trichloroethane, 1,1,2-trichloropropane, 1,1,2,2-tetrachloroethane, dibromomethane, chlorobenzene, chloroform, carbon tetrachloride, 1,1-dibromoethane, 1,2-dibromoethane, 1,1,1,2-tetrachloroethane and the like. The most preferred solvent is 1,1,1-trichloroethane.

The amount of solvent should be a solvent amount. This is an amount that will hold the 2,6-dialkyl-4-methoxymethylphenol and benzene compound in solution. A useful range is 500-1000 parts by weight inert solvent per each 100 parts of total benzene compound plus 2,6-dialkyl-4-methoxymethylphenol. In a more preferred embodiment, only part of the solvent (e.g., 25-50 weight percent) is placed in the reaction vessel together with the benzene compound and sulfuric or sulfonic acid. The remainder is used to dissolve the 2,6-dialkyl-4-methoxymethylphenol being added to the reactor.

The mole ratio of 2,6-dialkyl-4-methoxymethylphenol to benzene compounds depends on the number of 3,5-dialkyl-4-hydroxybenzyl groups to be introduced into the benzene compound. The moles of 2,6-dialkyl-4-methoxymethylphenol should be 100-130% of the stoichiometric amount. With durene the stoichiometric amount is 2 moles per mole of durene and with mesitylene the stoichiometric amount is 3 moles per mole of mesitylene. A preferred amount is 110-120% of the stoichiometric amount.

When sulfuric acid is used in the process, it is concentrated H₂SO₄. This includes concentrations of 75-100% and even oleums containing SO₃. A more preferred sulfuric acid catalyst is 80-98 weight percent H₂SO₄.

The amount of sulfuric acid is a catalytic amount. The prior art used about 0.9 mole H₂SO₄ per mole of 2,6-dialkyl-4-methoxymethylphenol. The amount of sulfuric acid is expressed in terms of active H₂SO₄ content. For example 100 grams of 98 weight percent sulfuric acid is 1.0 gram mole of H₂SO₄. The present process permits the use of less sulfuric acid than required by the prior art methods. A useful range is 0.05-2.0 moles H₂SO₄ per mole of 2,6-dialkyl-4-methoxymethylphenol. A more preferred amount is 0.07-1.0 mole H₂SO₄ and still more preferably 0.3-0.7 mole H₂SO₄ per mole of 2,6-dialkyl-4-methoxymethylphenol. A most preferred range is 0.4-0.5 mole H₂SO₄ per mole of 2,6-di-alkyl-4-methoxymethylphenol.

The reaction will proceed over a wide temperature range. A useful range in which to experiment is 10-150°C. A more preferred range is 20-100°C and a most preferred range is 20-50°C.

Suitable sulfonic acids for use in the process include both aliphatic and aromatic sulfonic acids. Some representative examples of aliphatic sulfonic acids are methane sulfonic acid, ethane sulfonic acid, propane sulfonic acid, trichloromethane sulfonic acid, trifluoromethane sulfonic acid and the like. Representative examples of aromatic sulfonic acids are benzene sulfonic acid, ortho, meta and para toluene sulfonic acid, chlorobenzene sulfonic acid, chlorotoluene sulfonic acid and the like. The more preferred sulfonic acids are methane sulfonic acid, ethane sulfonic acid and toluene sulfonic acids including mixtures thereof.

The amount of sulfonic acid is a catalytic amount. The prior art used about 3.1 moles toluene sulfonic acid per mole of 2,6-dialkyl-4-methoxymethylphenol. The present process permits the use of less sulfonic acid than required by the prior art methods. A useful range is 0.005-1.0 mole sulfonic acid per mole of 2,6-dialkyl-4-methoxymethylphenol. A more preferred amount is 0.01-0.5 mole sulfonic acid and still more preferably 0.015-0.1 mole sulfonic acid per mole of 2,6-dialkyl-4-methoxymethylphenol. A most preferred range is 0.03-0.05 mole sulfonic acid per mole of 2,6-dialkyl-4-methoxymethylphenol.

The reaction will proceed over a wide temperature range. A useful range in which to experiment is 0-150°C. A more preferred range is 10-80°C and a most preferred range is 15-40°C.

The reactor should be fitted to permit distillation of a methanol-containing distillate from the reaction mixture while feeding the 2,6-dialkyl-4-methoxymethylphenol. Depending on reaction temperature, it is usually necessary to lower the reactor pressure to cause the distillation. With a 1,1,1-trichloroethane solvent at 20°C, the solvent/methanol mixture distilled at 13.3 kPa (100 torr). Solvents having a normal boiling point above about 70°C are more efficient in removing methanol without with co-distilling a large amount of inert solvent.

The distillate removed can be merely discarded and replaced by the solvent being added with the 2,6-dialkyl-4-methoxymethylphenol solution. Preferably the distillate is treated to remove methanol and then recycled to the reaction mixture. One way to do this is to pass the distillate through an adsorbent which has an affinity for methanol. Zeolites can perform this function. Although both natural and synthetic zeolites can be used, the synthetic zeolites are preferred. The "A" type zeolites are effective, especially type 4A zeolite.

Better results are obtained when the 2,6-dialkyl-4-methoxymethylphenol is fed to the inert solvent containing the benzene compound and sulfuric or sulfonic acid catalyst over an extended period of time to prevent the accumulation of a large amount of 2,6-dialkyl-4-methoxymethylphenol in the reaction mixture. Feed time will depend upon scale and rate of methanol removal. A useful time range in which to experiment to optimize results is 0.5-12 hours. A more preferred feed period is 1-8 hours.

The following examples show the best modes known to the inventors for carrying out the process.

### Example 1

In a reaction flask fitted with a stirrer, thermometer, pressure equalized addition funnel and a reflux condenser was placed 35 mL of 1,1,1-trichloroethane, 1.17 g of mesitylene and 1.5 g of 95% sulfuric acid. The reflux condenser was constructed such that the condensate was drained down through a tube containing 25 g of activated type 4A zeolite and then returned to the reaction flask. The addition funnel was charged with a solution of 8.37 g of 2,6-di-tert-butyl-4-methoxymethylphenol in 40 mL of 1,1,1-trichloroethane. The stirred reaction flask was held at 20°C and the pressure in the system lowered to 13.3 kPa (100 torr). The 2,6-di-tert-butyl-4-methoxymethylphenol solution was added dropwise over a 3-hour period while maintaining a steady reflux stream draining through the zeolite bed and back into the reactor. Gas chromatograph analysis showed the reaction mixture excluding solvent to contain:

| | |
|---|---|
| 2,4,6-tri-(3,5-di-tert-butyl-4-hydroxybenzyl) mesitylene | 91.92% |
| 2,4-di-(3,5-di-tert-butyl-4-hydroxybenzyl) mesitylene | 0.80% |
| 4,4'-methylenebis(2,6-di-tert-butylphenol) | 7.27% |

Product can be recovered by distilling off part of the solvent and cooling the solution to crystallyze 2,4,6-tri-(3,5-di-tert-butyl-4-hydroxybenzyl) mesitylene.

### Example 2

In a glass reaction flask was placed 35 mL 1,1,1-trichloroethane, 0.08 g (0.0008 mole) of methane sulfonic acid and 1.18 g (0.01 mole) of mesitylene. The flask was equipped with a stirrer, thermometer, pressure equalized addition funnel and a reflux condenser that drained all condensate down through a bed of type 4A zeolite. The addition funnel was filled with a solution of 8.37 g (0.033 mole) 2,6-di-tert-butyl-4-methoxymethylphenol in 35 mL 1,1,1-trichloroethane. The stirred reaction mixture was warmed to 40°C and the pressure reduced to about 29.3 kPa (220 torr). Feed of 2,6-di-tert-butyl-4-methoxymethylphenol was started while a methanol-containing distillate formed. The condensed distillate was drained down through the bed of type 4A zeolite and back into the reaction flask. Feed time was two hours. During the feed, another 0.14 g of methane sulfonic acid was added in two portions (total added 0.22 g, 0.0013 mole). GC analysis of the final reaction mixture excluding solvent was

| | |
|---|---|
| 2,4,6-tri-(3,5-di-tert-butyl-4-hydroxybenzyl) mesitylene | 92.11% |
| 2,4-di-(3,5-di-tert-butyl-4-hydroxybenzyl) mesitylene | 1.39% |
| 4,4-methylenebis(2,6-di-tert-butylphenol) | 5.47% |

The product was recovered by crystallization and filtration.

## Claims

1. A process for making a compound having the formula 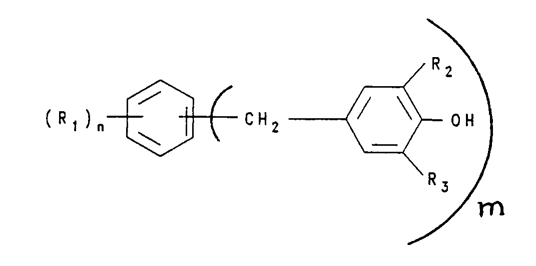 by feeding over a period of from 0.5 to 12 hours an aryl-substituted methoxy methyl reactant having the structure 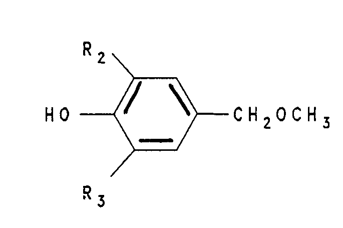 with a benzene compound having the structure 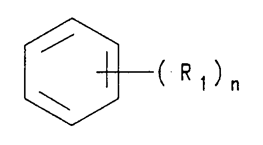 wherein R₁ is C₁₋₃ alkyl and R₂ and R₃ are independently selected from the group consisting of C₁₋₈ alkyl, C₅₋₈ cycloalkyl and C₇₋₁₂ aralkyl, n is zero or an integer from 1 to 4, m is an integer from 2 to 3, and m + n does not exceed 6, in an inert solvent in the presence of sulfuric acid or sulfonic acid at a temperature of 10-150°C and at a pressure such that the methanol formed during the reaction, distills out of the reaction mixture as a methanol-containing distillate, contacting said distillate with an absorbent having an affinity for methanol, thereby removing at least part of the methanol from said methanol-containing distillate and recycling the distillate resulting from the contact with said absorbent having an affinity for methanol.

2. A process of Claim 1 wherein said benzene compound is mesitylene.

3. A process of Claim 1or 2 wherein said reactant is 2,6-di-tert-butyl-4-methoxymethylphenol.

4. A process of any of the preceding claims wherein said inert solvent is a normally liquid chlorohydrocarbon having a normal boiling range of 40-200°C.

5. A process of any of claims 1-4 wherein said acid is sulfuric acid.

6. A process of any of claims 1-4 wherein said acid is an aromatic sulfonic acid.

7. A process of any of claims 1-4 wherein said acid is a C₁₋₂ alkane sulfonic acid.

8. The process according to Claim 1 wherein said compound is 1,4,6-tri(3,5-di-tert-butyl-4-hydroxybenzyl)mesitylene and said process further comprises, (a) placing a solvent amount of a chlorohydrocarbon having a normal boiling point of 40-150°C and 1 mole part of mesitylene and 0.05-0.2 mole part of a catalyst selected from methane sulfonic acid, ethane sulfonic acid or mixtures thereof in a reaction vessel, (b) feeding 3.1-3.4 mole parts of 2,6-di-tert-butyl-4-methoxymethylphenol to said reaction vessel over said extended period while the vessel contents are at a temperature of 20-60°C while maintaining said reaction vessel at a reduced pressure at which a methanol-containing distillate distills from the reaction mixture, and (c) recovering said 2,4,6-tri(3,5-di-tert-butyl-4-hydroxybenzyl)mesitylene.

9. The process according to claim 1 wherein said compound is 2,4,6-tri-(3,5-di-tert-butyl-4-hydroxybenzyl)mesitylene, said process further comprises
(a) placing a solvent amount of a normally liquid chlorohydrocarbon having a normal boiling range of 50-150°C in a reaction vessel, (b) adding about one mole part of mesitylene to said reaction vessel, (c) adding 0.25-2.0 moles of H₂SO₄ acid to said reaction vessel, (d) feeding 3-3.6 mole parts of 2,6-di-tert-butyl-4-methoxymethyl phenol to said reaction vessel over an extended period of 0.5-12 hours while maintaining the contents of said reaction vessel at 20-100°C and the pressure within said reaction such that methanol formed in the reaction distills out of said reaction vessel as a methanol-containing distillate and (e) recovering said 2,4,6-tri-(3,5-di-tert-butyl-4-hydroxybenzyl)mesitylene product.

10. A process of any of the preceding claims wherein said methanol-containing distillate is contacted with an adsorbent having an affinity for methanol thereby removing at least part of the methanol from said methanol-distillate and then recycling the methanol-depleted distillate to the reaction mixture.

## Patentansprüche

1. Verfahren zur Herstellung einer Verbindung der Formel 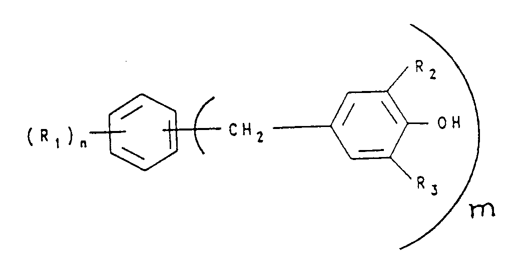 durch Einspeisen eines Aryl-substituierten Methoxymethylreaktanten der Struktur 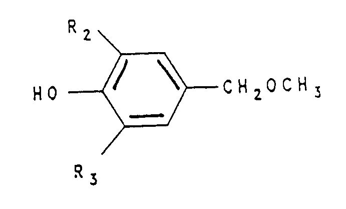 mit einer Benzolverbindung der Struktur 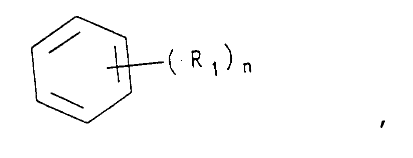 in der R₁ C₁₋₃-Alkyl und R₂ und R₃ unabhängig voneinander aus der aus C₁₋₈-Alkyl, C₅₋₈-Cycloalkyl und C₇₋₁₂-Aralkyl bestehenden Gruppe ausgewählt sind, n null oder eine ganze Zahl von 1 bis 4 ist, m eine ganze Zahl von 2 bis 3 ist und m + n sechs nicht übersteigt,
über einen Zeitraum von 0,5 bis 12 Stunden in ein inertes Lösungsmittel in Gegenwart von Schwefel- oder Sulfonsäure bei einer Temperatur von 10 bis 150°C und bei einem solchen Druck, daß das während der Reaktion gebildete Methanol als methanolhaltiges Destillat aus dem Reaktionsgemisch ausdestilliert,
In-Kontakt-bringen des Destillats mit einem Absorptionsmittel mit einer Affinität für Methanol, wodurch mindestens ein Teil des Methanols aus dem methanolhaltigen Destillat entfernt wird, und Rückführen des Destillats, das durch den Kontakt mit dem Absorptionsmittel mit einer Affinität für Methanol entstanden ist.

2. Verfahren nach Anspruch 1, bei dem die Benzolverbindung Mesitylen ist.

3. Verfahren nach Anspruch 1 oder 2, bei dem der Reaktant 2,6-Di-tert-butyl-4-methoxymethylphenol ist.

4. Verfahren nach einem der vorstehenden Ansprüche, bei dem das inerte Lösungsmittel ein normalerweise flüssiger Chlorkohlenwasserstoff mit einem normalen Siedebereich von 40 bis 200°C ist.

5. Verfahren nach einem der Ansprüche 1 bis 4, bei dem die Säure Schwefelsäure ist.

6. Verfahren nach einem der Ansprüche 1 bis 4, bei dem die Säure eine aromatische Schwefelsäure ist.

7. Verfahren nach einem der Ansprüche 1 bis 4, bei dem die Säure eine C₁₋₂-Alkansulfonsäure ist.

8. Verfahren nach Anspruch 1, bei dem die Verbindung 1,4,6-Tri(3,5-di-tert-butyl-4-hydroxybenzyl)mesitylen ist und das Verfahren zusätzlich folgende Schritte umfaßt:
(a) Einbringen einer lösend wirkenden Menge eines Chlorkohlenwasserstoffs mit einem normalen Siedepunkt von 40 bis 150°C, 1 Molteil Mesitylen und 0,05 bis 0,2 Molteilen eines aus Methansulfonsäure, Ethansulfonsäure oder Mischungen davon ausgewählten Katalysators in ein Reaktionsgefäß,
(b) Einspeisen von 3,1 Molteilen 2,6-Di-tert-butyl-4-methoxymethylphenol über den ausgedehnten Zeitraum in das Reaktionsgefäß, während dessen Inhalt sich bei einer Temperatur von 20 bis 60°C gehalten wird, wobei das Reaktionsgefäß auf einem verringerten Druck gehalten wird, bei dem ein methanolhaltiges Destillat aus der Reaktionsmischung ausdestilliert, und
(c) Rückgewinnung des 2,4,6-Tri(3,5-di-tert-butyl-4-hydroxybenzyl)mesitylens.

9. Verfahren nach Anspruch 1, bei dem die Verbindung 2,4,6-Tri(3,5-di-tert-butyl-4-hydroxybenzyl)mesitylen ist und das zusätzlich folgende Schritte umfaßt:
(a) Einbringen einer lösend wirkenden Menge eines normalerweise flüssigen Chlorkohlenwasserstoffs mit einem normalen Siedebereich von 50 bis 150°C in ein Reaktionsgefäß;
(b) Zugabe von etwa 1 Molteil Mesitylen in das Reaktionsgefäß;
(c) Zugabe von 0,25 bis 2,0 Mol H₂SO₄ zum Reaktionsgefäß,
(d) Einspeisen von 3 bis 3,6 Molteilen 2,6-Di-tert-butyl-4-methoxymethylphenol in das Reaktionsgefäß über einen ausgedehnten Zeitraum von 0,5 bis 12 Stunden, während dem der Inhalt des Reaktionsgefäßes bei 20 bis 100°C gehalten und der Druck innerhalb der Reaktion so eingestellt wird, daß in der Reaktion gebildetes Methanol als methanolhaltiges Destillat aus dem Reaktionsgefäß ausdestilliert, und
(e) Rückgewinnung des 2,4,6-Tri(3,5-di-tert-butyl-4-hydroxybenzyl)mesitylenprodukts.

10. Verfahren nach einem der vorstehenden Ansprüche, bei dem man das methanolhaltige Destillat mit einem Adsorptionsmittel mit einer Affinität für Methanol in Kontakt bringt, wodurch mindestens ein Teil des Methanols aus dem Methanoldestillat entfernt wird, und das an Methanol verarmte Destillat zum Reaktionsgemisch zurückführt.

## Revendications

1. Procédé de préparation d un composé de formule 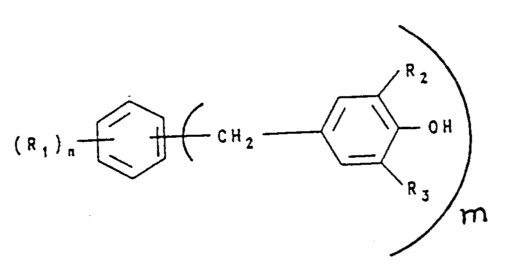 en fournissant sur une période de 0,5 à 12 heures un réagissant de méthoxy méthyle à substitution aryle, de structure 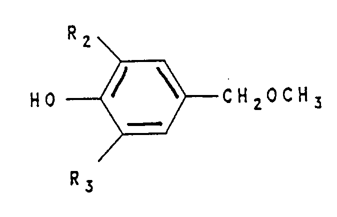 avec un composé du benzène de structure 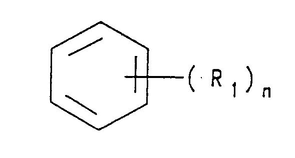 où R₁ représente un alkyle en C₁₋₃ et R₂ et R₂ sont choisis indépendamment dans le groupe formé d'alkyle en C₁₋₈, de cycloalkyle en C₅₋₈ et d'aralkyle en C₇₋₁₂, n vaut zéro ou un entier de 1 à 4, m est un entier valant 2 à 3, et m + n ne dépasse pas 6, dans un solvant inerte en présence d'acide sulfurique ou d'acide sulfonique, à une température de 10-150°C et sous une pression telle que le méthanol formé pendant la réaction sort par distillation du mélange réactionnel sous la forme d'un distillat contenant du méthanol, en mettant en contact ledit distillat avec un adsorbant présentant une affinité pour le méthanol, pour ainsi éliminer au moins une partie du méthanol dudit distillat contenant du méthanol, et en recyclant ledit distillat résultant du contact avec ledit adsorbant présentant une affinité pour le méthanol.

2. Procédé selon la revendication 1, dans lequel ledit composé du benzène est le mésitylène.

3. Procédé selon la revendication 1 ou 2, dans lequel ledit réagissant est le 2,6-di-tert-butyl-4-méthoxyméthylphénol.

4. Procédé selon l'une quelconque des revendications précédentes, dans lequel ledit solvant inerte est un hydrocarbure chloré normalement liquide présentant un intervalle normal d'ébullition de 40-200°C.

5. Procédé selon l'une quelconque des revendications 1-4, dans lequel ledit acide est l'acide sulfurique.

6. Procédé selon l'une quelconque des revendications 1-4, dans lequel ledit acide est un acide sulfonique aromatique.

7. Procédé selon l'une quelconque des revendications 1-4, dans lequel ledit acide est un acide (alcane en C₁₋₂) sulfonique.

8. Procédé selon la revendication 1, dans lequel ledit composé est le 1,4,6-tri(3,5-di-tert-butyl-4-hydroxybenzyl)mésitylène et ledit procédé comprend de plus les étapes consistant à (a) placer une quantité solvante d'un hydrocarbure chloré présentant un point d'ébullition normal de 40-150°C et 1 partie molaire de mésitylène et 0,05-0,2 partie molaire d'un catalyseur choisi parmi l'acide méthane sulfonique, l'acide éthane sulfonique ou des mélanges de ceux-ci dans un réacteur, (b) amener dans ledit réacteur 3,1-3,4 parties molaires de 2,6-di-tert-butyl-4-méthoxyméthylphénol sur ladite période prolongée, alors que le contenu du reacteur se trouve a une température de 20-60°C, tout en maintenant le réacteur sous une pression réduite à laquelle un distillat contenant du méthanol sort par distillation du mélange réactionnel, et (c) récupérer ledit 2,4,6-tri(3,5-di-tert-butyl-4-hydroxybenzyl)mésitylène.

9. Procédé selon la revendication 1, dans lequel ledit composé est le 2,4,6-tri-(3,5-di-tert-butyl-4-hydroxybenzyl)mésitylène, ledit procédé comprenant de plus les étapes consistant à:
(a) placer dans un réacteur une quantité solvante d'un hydrocarbure chloré normalement liquide présentant un intervalle d'ébullition normal de 50-150°C, (b) ajouter environ une partie molaire de mésitylène audit réacteur, (c) ajouter 0,25-2,0 moles d'acide H₂SO₄ audit réacteur, (d) amener dans ledit réacteur 3-3,6 parties molaires de 2,6-di-tert-butyl-4-méthoxyméthylphénol sur une période prolongée, de 0,5-12 heures, tout en maintenant le contenu du réacteur à 20-100°C, et la pression dans la réaction telle que le méthanol formé au cours de la réaction quitte par distillation ledit réacteur sous la forme d'un distillat contenant du méthanol, et (e) récupérer ledit produit de 2,4,6-tri(3,5-di-tert-butyl-4-hydroxybenzyl) mésitylène.

10. Procédé selon l'une quelconque des revendications précédentes, dans lequel ledit distillat contenant du méthanol est mis en contact avec un adsorbant présentant une affinité pour le méthanol, pour ainsi éliminer au moins une partie du méthanol du distillat contenant du méthanol, et recycler ensuite le distillat appauvri en méthanol du mélange réactionnel.
